# FASCICULE DE BREVET EUROPEEN

(11) **EP 2 713 942 B1**
(45) Date de publication et mention de la délivrance du brevet: **12.07.2017**
(21) Numéro de dépôt: 12731041.5
(22) Date de dépôt: 22.05.2012
(51) Int. Cl.: A61F 2/00, D04B 21/12

(54) **IMPLANT TEXTILE**
TEXTILIMPLANTAT
TEXTILE IMPLANT

(30) Priorité: 23.05.2011 FR 1154473
(43) Date de publication de la demande: 09.04.2014
(73) Titulaire: MDB Texinov SA, 38110 Saint Didier de la Tour (FR); Deltaval, 69006 Lyon (FR)
(72) Inventeur: TANKERE, Jacques, F-01800 Meximieux (FR); DUCOL, Jean-Paul, F-69170 Les Sauvages (FR); CHAPUIS, Christian, F-38300 Bourgoin Jallieu (FR); MIGNOT, Eric, F-38620 Velanne (FR); SIMONS, Damien, F-69007 Lyon (FR)
(74) Mandataire: Cabinet Laurent & Charras
(86) Numéro de dépôt international: PCT/FR2012/051147
(87) Numéro de publication internationale: WO 2012/160309

(56) Documents cités:
- EP-A1- 1 600 118
- WO-A2-03/028585
- FR-A1- 2 859 624
- FR-A1- 2 941 145
- US-A1- 2003 220 538
- US-A1- 2011 021 868

## Description

### DOMAINE DE L'INVENTION

L'invention concerne le domaine des implants textiles, plus particulièrement destinés à être mis en oeuvre dans le cadre du traitement de l'incontinence, tant chez l'homme que chez la femme, mais également du prolapsus génital ou encore des hernies.

En l'espèce, l'implant permanent de l'invention est constitué d'un tricot ajouré réalisé selon la technique bien connue de la maille jetée, séparable en implants unitaires dans la largeur par processus de démaillage de chaînette ou par découpe partielle sur métier.

L'implant de l'invention présente une largeur variable, et est susceptible en outre d'intégrer des bretelles de soutien ou de fixation multiples.

### ETAT ANTERIEUR DE LA TECHNIQUE

Les implants du type en question sont aujourd'hui largement développés. Ils ont vocation à soutenir un organe tel que l'utérus, la vessie, ou le rectum, susceptibles de se trouver non soutenus en raison de la déficience d'un ensemble de muscles, de ligaments et/ou de fibres.

Le prolapsus génital est un déplacement anormal d'un ou plusieurs organes du pelvis féminin vers le bas. Ce dysfonctionnement du trouble de la statique pelvienne est communément appelée « descente d'organes ». Il est responsable d'un cinquième des indications de chirurgie gynécologique. En effet la médecine « douce » qui consiste en la rééducation périnéale permet de maintenir les prolapsus débutants, cependant, une fois que le prolapsus est avancé (stade 2 ou 3), elle ne permet pas de réintégrer les organes qui sont descendus.

Il doit donc être envisagé dans ces circonstances un traitement chirurgical consistant à remplacer les moyens de suspension (fascias, ligaments) ou les moyens de soutènement (muscles du périnée) devenus défaillants.

Bien que plus fréquent chez les femmes (on estime que 25 à 30 % des femmes entre 45 et 85 ans en souffrent, avec un pic de fréquence entre 60 et 80 ans) le prolapsus génital touche aussi les hommes notamment quand l'homme a eu un cancer du rectum ou de la prostate, dont le traitement passe par une intervention chirurgicale.

Le cancer de la prostate est le type de cancer le plus fréquent chez l'homme : on estime que 1 homme sur 6 vivant jusqu'à 80 ans est appelé à le développer. Dans environ 4% des cas, le patient subit des complications se traduisant par de l'incontinence urinaire.

Le chirurgien intervient soit par voie haute (voie abdominale), soit par voie basse (voie vaginale) ou par laparoscopie sans ouverture, pour fixer un ruban de soutien qui permet d'accrocher l'utérus ou la vessie.

Parmi les dispositifs mis en oeuvre pour traiter les patients, on connaît par exemple les implants du type bandelette. Il existe principalement deux types de bandelette, respectivement :
- l'un dénommé TVT (pour l'acronyme anglo-saxon « Tension-Free Vaginal Tape »), constitué d'un ruban réalisé en polypropylène tricoté, mis en place par voie rétro-pubienne ; et
- l'autre dénommé TOT (pour l'acronyme anglo-saxon « TransObturator Tape »), constitué également d'une bandelette, mise en place par traitement chirurgical par voie trans-obturatrice.

On a décrit dans le document EP 1 342 450 une méthode d'implantation, dite « par voie trans-obturatrice » - d'un tel implant de soutien. Elle consiste à positionner une bandelette sous l'urètre afin de maintenir ce canal en suspension. L'urètre repose alors sur la partie centrale de la bandelette, elle-même maintenue mécaniquement à ses extrémités par passage dans les trous obturateurs de l'os iliaque. Ainsi positionnée, la bandelette permet de pallier le dysfonctionnement du système ligamento-musculaire qui assure la fermeture du canal de l'urètre. Cette méthode présente moins de risques de perforation d'organes que la voie rétro-pubienne. L'implant ainsi accroché peut être fixé par voie de suture, d'agrafe ou par ancrage tissulaire.

L'ancrage tissulaire est par ailleurs en cours de développement. A cet égard, le document WO2004/091443 décrit un maintien principalement par friction entre l'implant et le tissu traversé.

En outre, le document WO2010/023418 décrit un implant prothétique de soutènement sous-urétral comprenant un gousset à chacune de ses extrémités. La méthode d'implantation décrite est avantageuse pour le patient car elle évite les risques de perforation vésicale et ne nécessite aucune fixation osseuse.

De manière générale, un tel implant de soutien destiné à l'urologie féminine doit posséder une résistance mécanique suffisante pour assurer le maintien des organes, et en particulier dans le sens longitudinal de la bandelette.

Il a ainsi été montré que la bandelette constitutive de l'implant doit avoir préférentiellement une largeur stable à l'emplacement du contact avec l'urètre ou l'organe à maintenir afin d'éviter toute douleur pour le patient. En effet, si l'extensibilité d'un tricot dans le sens longitudinal est permise au niveau de ce contact, celle-ci entraîne une réduction systématique de la dimension du tricot dans le sens de sa largeur, susceptible de provoquer une douleur à la hauteur de l'urètre.

On observe fréquemment que les implants ont tendance à se rétracter, qu'ils ne présentent pas de stabilité spatiale optimale et donc qu'ils s'adaptent mal à l'anatomie du patient. Afin d'éviter ce phénomène, on a proposé, par exemple dans le document US 2010/261950, une bandelette présentant un système de réglage de la traction afin que l'effort transmis à la bandelette ne provoque pas de changement dimensionnel.

S'agissant de l'incontinence urinaire chez l'homme, la morphologie masculine est telle que l'urètre est entouré de corps spongieux et de corps caverneux disposés de part et d'autre du corps spongieux. Cet environnement, sensiblement différent de celui de la femme, implique une surface de contact avec les organes à soutenir plus importante. Aussi, un maintien ferme de l'urètre n'est possible que si la bandelette présente une stabilité dimensionnelle dans le sens longitudinal, qui se traduit par une déformation à la contrainte faible.

Les implants proposés à ce jour ne donnent pas satisfaction à cet égard. Ainsi, le document US 2009/0192346 décrit une bandelette à largeur variable, découpée dans un tricot homogène, dont la trame est régulière sur l'ensemble de la surface. Ce système ne permet pas de fait de gérer les caractéristiques mécaniques différentes nécessaires sur la partie support plus large et sur la partie bretelle de fixation.

On a également décrit, par exemple dans le document FR 2 919 996, la technique de l'implantation par voie trans-obturatrice. Cette technique propose une adaptation sur l'homme de la technique d'implantation initialement développée chez la femme. Il est ainsi mentionné l'assemblage de deux bandelettes utilisées en urologie féminine, jointes par couture sur leur bord et dans leur partie centrale, dans le but d'obtenir une bandelette dont la largeur serait doublée en son centre. Dans ce cas, les bandelettes sont laissées libres en leurs extrémités afin de permettre la fixation de l'implant dans les trous obturateurs de l'os iliaque. Cet implant présente cependant une largeur utile limitée en son centre, au maximum égale à deux fois la largeur d'une bandelette utilisée en urologie féminine, à savoir 2 à 3 cm maximum. Il apparaît clairement souhaitable que cette surface soit adaptée à la morphologie du patient et que la bandelette puisse atteindre une largeur de 5 à 6 cm.

En outre, la mise en oeuvre d'une couture reliant les deux bandelettes en leurs bords induit une modification de la structure de l'implant, entrainant inévitablement un risque de faiblesse mécanique au niveau du centre de la bandelette. Une couture qui cèderait sous la contrainte du poids des organes nécessiterait une nouvelle intervention, et pourrait être à l'origine de complications. Cette couture, réalisée lors d'une deuxième étape de fabrication, complique en outre la géométrie de la bandelette et peut créer un relief en surface de la bandelette, qui n'est pas souhaitable et peut s'avérer douloureux pour le patient.

Le document US2011/021868 décrit un implant constitué par une bandelette présentant une largeur variable, obtenue par variation de la tension des fils qui la constituent. Le document EP-A-1600118 décrit une autre implant de tricot pour le traitement de l'incontinence. Enfin, il peut être souhaitable de disposer de propriétés mécaniques différentes ou un comportement mécanique différent entre le centre de l'implant et ses extrémités. Plus spécifiquement, on peut souhaiter disposer une bandelette dont le comportement mécanique est bloqué au centre de l'implant et ce, dans les deux directions, et corollairement rechercher un allongement maîtrisé, voire une certaine élasticité au niveau des extrémités de la bandelette. En effet, dans le cas du traitement de pathologies d'incontinence d'effort ou de prolapsus, on recherche un comportement mécanique proche du comportement ligamento-musculaire déficient. De ce fait, la recherche d'un comportement de type visco-élastique proche de celui des ligaments est judicieuse sur la partie latérale de la bandelette, tandis que l'on cherche une partie centrale exempte de toute déformation sous la contrainte, afin d'éviter toute douleur au niveau de l'urètre ou des corps spongieux. Par voie de conséquence, il apparaît clairement que les propriétés requises sur la partie centrale de la bandelette sont différentes des caractéristiques attendues sur ses extrémités. C'est ainsi que selon la nature des tissus, l'âge du patient, sa morphologie, la nature de la pathologie traitée, le poids des organes à supporter ou la méthode retenue pour l'implantation, on peut être amené à exiger des propriétés mécaniques différentes tout au long de la bandelette. Traditionnellement, ces différents implants sont destinés à demeurer dans le corps humain de manière définitive. Partant, ils sont constitués de matériaux synthétiques ou naturels biocompatibles afin de permettre leur acceptation par les tissus humains, et comprennent une base en matériau non résorbable.

En outre, si l'augmentation de la quantité de matière du tricot constitutif de l'implant a pour effet d'améliorer sa résistance mécanique, en contrepartie, elle tend à diminuer l'acceptation dudit tricot dans les tissus humains. Ainsi, la quantité de matière que compose le tricot, que l'on peut évaluer en mesurant le poids au mètre carré du tricot, doit être optimisée afin de respecter les contraintes mécaniques de l'implant et les contraintes d'intégration tissulaire dudit tricot. Il est donc admis que la structure ajourée macroporeuse d'un tel tricot facilite sont intégration dans les tissus humains et la colonisation des cellules.

Les bandelettes en question peuvent être obtenues par la découpe de tricots dans le sens longitudinal au moyen de couteaux, de ciseaux ou de tout autre moyen traditionnel de découpe. Or, ces moyens de découpe génèrent la présence de particules de matière polymérique, susceptibles de se trouver libres dans les tissus humains après implantation. On parle alors de phénomène de relargage, très néfaste à la bonne intégration du tricot dans le corps humain.

Afin de pallier cet inconvénients, on a proposé, par exemple dans le document FR 2 884 835 un tricot démaillable, susceptible de permettre la séparation d'un tel tricot en une pluralité de bandelettes. Ce système consiste à mettre en oeuvre un fil décrivant une armure de type chainette entre les différentes bandelettes que l'on souhaite ainsi définir. La simple traction de ce fil libère alors lesdites bandelettes, permettant ainsi de s'affranchir de tout phénomène de relargage.

Le besoin s'est donc fait sentir d'un implant à propriétés mécaniques ou comportement mécanique différencié et susceptible également de variations dimensionnelles en largeur.

### EXPOSE DE L'INVENTION.

Ainsi donc, la présente invention propose un implant, plus particulièrement destiné au traitement de l'incontinence urinaire masculine ou féminine, constitué d'une bandelette réalisée en un tricot unitaire à base de matériau(x) biocompatible(s), présentant une largeur susceptible de varier le long de sa dimension principale, et pourvue au moins au niveau de l'une de ses extrémités d'au moins une bretelle de fixation, émanant directement de sa structure propre. Ledit tricot étant obtenu par la technologie à mailles jetées sur un métier chaine ou RACHEL mettant en oeuvre des aiguilles automatiques ou carabine, lesdites aiguilles n'étant pas fournies d'un ou plusieurs fils pendant au moins une rangée et ce, sans interrompre le mécanisme de formation de la maille.

En d'autres termes, l'invention propose un implant tricoté, qui nonobstant le caractère unitaire de sa structure, est susceptible de présenter des variations dimensionnelles et des divisions aptes à constituer une ou plusieurs bretelles de fixation, et ce, sans observer de variation de la dimension des mailles qui le constituent. Ainsi donc, la ou les variations dimensionnelles de l'implant de l'invention ne résultent pas d'une quelconque variation de la tension des fils qui le constituent.

Selon une caractéristique avantageuse de l'invention, cet implant, bien que présentant une largeur variable, est exempt de toutes extrémités libres de fils qui le constituent. En d'autres termes, les bords de l'implant sont systématiquement constitués de boucles de fils. Ce faisant, on s'affranchit d'une part, des phénomènes d'irritation, fréquemment observés avec les implants de l'art antérieur, et d'autre part, on évite les risques de relargage de particules ou d'éléments constitutifs desdits implants.

En outre, et selon une caractéristique avantageuse de l'invention, les propriétés mécaniques de la bandelette peuvent varier le long de l'implant, et notamment des bretelles de fixation par rapport à la zone centrale, servant traditionnellement de zone support d'un organe. Ainsi, les propriétés d'extensibilité ou d'élasticité et de résistance mécanique sont plus ou moins développées le long de la bandelette.

L'implant de l'invention est, comme déjà dit, constitué d'un tricot. Selon l'invention, ce tricot est obtenue par la technologie maille jetée, encore appelée tricotage chaîne ou Rachel. Ce faisant, on dispose d'un tricot souple, susceptible de pouvoir être bloqué, par exemple au centre de l'implant, c'est-à-dire au niveau de la zone de soutien de l'organe, et extensible dans le sens longitudinal et/ou dans le sens travers, selon le besoin.

Du fait de la maitrise de cette technologie de tricotage, différents modèles peuvent être développés pour répondre à des populations cibles (taille des patients) ou à des besoins pathologiques spécifiques (taille des prolapsus).

En outre, et selon l'invention, les aiguilles mises en oeuvre sur le métier chaine ou Rachel ou à crochets sont de type dit « automatique », également dénommées dans le domaine considéré par aiguilles « carabine ». En effet, il est possible grâce à ces aiguilles de ne pas fournir une ou plusieurs aiguilles d'un ou plusieurs fils pendant au moins une rangée et ce, sans interrompre le mécanisme de formation de la maille précédemment décrit, et donc, sans risque de démaillage du tricot en résultant.

De manière connue, et contrairement aux aiguilles à clapet traditionnellement mises en oeuvre dans la technologie du tricotage à maille jetée, ces aiguilles automatiques sont toujours ouvertes, l'entrée du fil dans l'aiguille résultant du glissement dudit fil le long du fût de l'aiguille, jusqu'à une fente qui laisse passer le fil dans l'aiguille. Le fil ne peut entrer dans l'aiguille que s'il présente un angle qui correspond au positionnement de la fente sur l'aiguille. Ainsi, il n'est pas indispensable de fermer l'aiguille à l'aide d'une rangée de mailles précédemment formées, comme c'est le cas avec des aiguilles à clapet, de sorte qu'il devient possible de modifier la largeur du tricot en raison de la technique de tricotage mise en oeuvre, sans nécessiter de découpe ultérieure. Les bords libres du tricot ne sont donc plus constitués que de boucles, en l'absence de toute extrémité de fil.

Comme déjà précisé, l'implant de l'invention présente une structure unitaire. En d'autres termes, sa fabrication ne nécessite aucune couture ou autre opération supplémentaire consistant à rapporter des parties ou éléments additionnels sur le tricot. Ainsi la ou les bretelles dont il est muni ne sont pas rapportées, mais résultent directement de l'opération de tricotage. Nonobstant ce caractère unitaire, l'implant de l'invention peut présenter une largeur et des propriétés mécaniques spécifiques dans sa partie centrale, la largeur et les performances mécaniques pouvant corollairement être différentes entre le centre et les extrémités de l'implant.

L'implant peut être mis en place manuellement ou à l'aide d'instruments non contendants, par voie vaginale ou abdominale ou laparotomie. Le système de fixation peut reposer sur la technologie de suture mais également tout système d'ancrage tissulaire.

Avantageusement, le tricot souple ajouré constitutif de l'implant est composé de plusieurs nappes de fils de nature polymériques synthétiques ou naturels biocompatibles résorbables ou non liées entre elles.

En fonction de l'armure sélectionnée pour la réalisation du tricot, c'est-à-dire du liage particulier de ces nappes de fils entre elles, et donc de l'agencement de ces fils les uns par rapport aux autres pour former une surface plane, on dispose d'une densité de mailles, autrement dit, d'un nombre de mailles au centimètre dans le sens de la production du métier variable et adaptable au gré des besoins.

De ce fait, il devient possible de disposer d'une largeur variable et/ou d'un comportement mécanique spécifique le long de la bandelette, en changeant d'armure et/ou en changeant la densité de maille.

On peut ainsi souhaiter disposer de caractéristiques de blocage mécanique de la bandelette dans le sens production du métier à tricoter, ainsi que dans le sens trame.

Par exemple, on peut choisir comme armure cette connue sous le nom de « chaînette tramée », qui possède des caractéristiques de déformation dans le sens de la longueur

### DESCRIPTION DES DESSINS

La manière dont l'invention peut être réalisée et les avantages qui en découlent, ressortiront mieux des exemples de réalisation qui suivent, donnés à titre indicatif et non limitatif à l'appui des figures annexées.
La figure 1 est une représentation schématique d'un implant conforme à l'invention, mettant en oeuvre une structure textile tricotée comportant deux largeurs différentes directement obtenues en production.
La figure 2 est une représentation schématique d'un implant conforme à une autre forme de réalisation de l'invention.

### DESCRIPTION DETAILLEE DE L'INVENTION

Comme indiqué en préambule, l'objet de l'invention consiste en la réalisation d'implants à largeur variable pour répondre aux fonctions spécifiques de chacune des parties qui les constituent.

Un tel implant ou bandelette comporte d'une part une partie support de l'organe qui est à protéger, à soutenir ou à maintenir dans une certaine position, et d'autre part, une ou deux autres parties dont la fonction réside dans l'accrochage ou la fixation dans le corps du patient.

La partie centrale - support de l'organe est traditionnellement plus large que les parties bretelles de fixation. Il peut cependant être envisagé d'autres cas de figure, dans lesquels cette configuration est inversée.

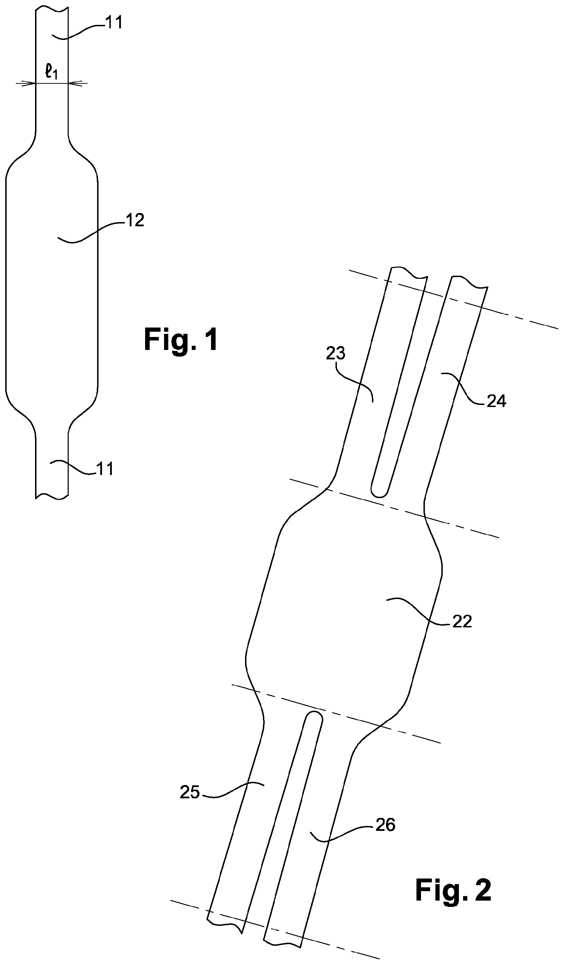

Ainsi, l'implant de la figure 1 est constitué d'un tricot réalisé à base de polymère biocompatible, et par exemple, de fils de polypropylène.

Ce tricot présente une zone centrale **12** de laquelle émanent deux zones extrêmes **11** de largeur 11 plus réduite. La zone centrale **12** est destinée à faire fonction de zone support de l'organe à soutenir, et par exemple l'urètre. Les deux zones extrêmes **11** permettent de remplir la fonction de fixation.

La figure 2 représente une autre forme de réalisation de l'implant - objet de l'invention. En l'espèce, la zone centrale **22,** assurant là encore la fonction de support et de maintien d'un organe, se prolonge par des bretelles de fixation, respectivement **23** et **24** d'un côté, et **25** et **26** de l'autre.

Comme on peut l'observer, il n'existe aucune discontinuité entre les bretelles de fixation et la zone centrale, l'ensemble constituant un tricot unitaire, obtenu sur un métier de type chaine ou Rachel ou encore à crochets.

Selon une caractéristique avantageuse de l'invention, les bords libres des bretelles de fixation **23 - 26** sont exempts de tout effilochage des bords, et permettant de s'affranchir de tous risques de relargage de partie de l'implant dans le corps. A cet effet, on utilise au sein du métier à tricoter mis en oeuvre des aiguilles automatiques (aiguilles carabines).

Avec de telles aiguilles, les mailles se forment successivement grâce à la géométrie de l'aiguille considérée et le positionnement judicieux du fil, présentant, comme déjà indiqué, un angle déterminé par rapport au fût de l'aiguille.

Ce faisant, il est possible en utilisant de telles aiguilles, de réaliser un tricot sans fournir de manière systématique et impérative à chaque aiguille au moins un fil à chaque rangée de maille. En d'autres termes, il est possible de fournir une aiguille avec un ou plusieurs fils pendant N1 rangées de mailles, puis de ne plus fournir cette aiguille pendant N2 rangées pour enfin réalimenter l'aiguille avec un ou plusieurs fils pendant N3 rangées sans observer de démaillage du tricot.

Ainsi donc, de par l'utilisation d'aiguilles dites « automatiques », il devient possible de réaliser des tricots dont la largeur varie le long de sa production selon que l'on fournit ou non certaines aiguilles d'un ou plusieurs fils. Cette faculté nécessite un arrangement de fil particulier et une armure judicieusement étudiée, que l'homme du métier est capable de déterminer en fonction des besoins sollicités en termes d'importance de la variation de largeur souhaitée.

En outre, les tensions de chaque nappe de fils sont régulées de manière séquentielle pour permettre la fabrication dudit implant.

Dans l'exemple décrit, la zone centrale de maintien **22** présente une longueur comprise entre 40 et 120 mm, avantageusement 90 mm.

Corollairement, les bretelles de fixation **23 - 26** présentent une longueur comprise entre 100 et 200 mm, avantageusement 150 mm.

Quel que soit l'implant (de la figure 1 ou de la figure 2), on dispose d'une largeur variable le long de sa dimension principale.

On peut également obtenir par simple changement d'armures ou de densité des variations du comportement mécanique, notamment en termes d'extensibilité ou d'élasticité.

L'implant ainsi obtenu est directement utilisable par le praticien lors de l'opération, sans nécessiter une quelconque découpe pour définir par exemple les bretelles.

## Revendications

1. Implant, plus particulièrement destiné au traitement de l'incontinence urinaire masculine ou féminine, constitué d'une bandelette réalisée en un tricot unitaire présentant une largeur variable le long de sa dimension principale, et pourvue au moins au niveau de l'une de ses extrémités d'au moins une bretelle de fixation (11, 22 - 26), émanant directement de sa structure propre, ledit tricot étant obtenu par la technologie à mailles jetées sur un métier chaine ou RACHEL mettant en oeuvre des aiguilles automatiques ou carabine, lesdites aiguilles n'étant pas fournies d'un ou plusieurs fils pendant au moins une rangée et ce, sans interrompre le mécanisme de formation de la maille.

2. Implant selon la revendication 1, ***caractérisé* en ce que** les propriétés mécaniques de la bandelette varient le long de la dimension principale de l'implant, et notamment des bretelles de fixation (11, 22 - 26) par rapport à la zone centrale, servant traditionnellement de zone support d'un organe.

3. Implant selon la revendication 2, ***caractérisé* en ce que** les propriétés mécaniques variables sont l'extensibilité et la résistance mécanique.

4. Implant selon l'une des revendications 1 à 3, ***caractérisé* en ce que** les bords latéraux du tricot sont limitativement constitués de boucles, à l'exclusion de toute extrémité libre de l'un quelconque des fils qui le constituent.

5. Implant selon l'une des revendications 1 à 4, ***caractérisé* en ce que** le tricot qui le constitue est obtenue par la technologie maille jetée sur métier chaine, Rachel ou à crochets, et mettant en oeuvre des aiguilles automatiques.

6. Implant selon la revendication 5, ***caractérisé* en ce que** le tricot est bloqué au niveau de la zone centrale de soutien ou de maintien (12, 22), et est extensible dans le sens longitudinal et/ou dans le sens travers, selon le besoin.

7. Implant selon l'une des revendications 1 à 6, ***caractérisé* en ce qu'**il comprend au moins deux bretelles de fixation émanant de part et d'autre de la zone centrale de maintien ou de soutien.

8. Implant selon l'une des revendications 1 à 7, ***caractérisé* en ce que** le tricot qui le constitue est exempt de toute couture.

9. Implant selon l'une des revendications 1 à 8, ***caractérisé* en ce que** le tricot qui le constitue est composé de plusieurs nappes de fils de nature polymériques synthétiques ou naturels biocompatibles résorbables ou non liées entre elles.

## Patentansprüche

1. Implantat, insbesondere zur Behandlung von männlicher oder weiblicher Harninkontinenz, das aus einem Band besteht, das aus einem integralen Gewirk ausgeführt ist, dessen Breite entlang der Hauptdimension variabel ist, und das an mindestens einem seiner Endstücke mit mindestens einem Befestigungsriemen (11, 22 - 26) versehen ist, der direkt aus der eigentlichen Struktur hervorgeht, wobei dieses Gewirk durch Kettenwirk-Technologie auf einer Kettenwirk- oder RASCHEL-Maschine hergestellt wird, auf der automatische oder Karabiner-Nadeln eingesetzt werden, wobei in mindestens einer Reihe diese Nadeln nicht mit einem oder mehreren Fäden beliefert werden und zwar ohne den Mechanismus der Maschenbildung zu unterbrechen.

2. Implantat gemäß Anspruch 1, ***dadurch gekennzeichnet, dass*** die mechanischen Eigenschaften des Bandes über die Länge der Hauptdimension des Implantats variieren und insbesondere die der Befestigungsriemen (11, 22 - 26), bezogen auf die zentrale Zone, die üblicherweise zum Stützen eines Organs dient.

3. Implantat gemäß Anspruch 2, ***dadurch gekennzeichnet, dass*** die variablen mechanischen Eigenschaften des Bandes die Dehnbarkeit und die mechanische Festigkeit sind.

4. Implantat gemäß einem der Ansprüche 1 bis 3, ***dadurch gekennzeichnet, dass*** die Seitenränder des Gewirks abgrenzend aus Schlaufen bestehen, unter Ausschluss jedes freien Endstücks irgendeines der Fäden, aus denen sie bestehen.

5. Implantat gemäß einem der Ansprüche 1 bis 4, ***dadurch gekennzeichnet, dass*** das Gewirk, aus denen es besteht, durch Kettenwirk-Technologie auf Kettenwirk-, Raschel- oder Platinenmaschinen, bei dem automatische Nadeln eingesetzt werden, hergestellt wird.

6. Implantat gemäß Anspruch 5, ***dadurch gekennzeichnet, dass*** das Gewirk in Höhe der zentralen Stütz- oder Haltezone blockiert wird (12, 22), und, je nachdem was notwendig ist, in Längsrichtung und/oder in Querrichtung dehnbar ist.

7. Implantat gemäß einem der Ansprüche 1 bis 6, ***dadurch gekennzeichnet, dass*** es mindestens zwei Halteriemen enthält, die beiderseits der zentralen Stütz- oder Haltezone herausführen.

8. Implantat gemäß einem der Ansprüche 1 bis 7, ***dadurch gekennzeichnet, dass*** das Gewirk, aus dem es besteht, keinerlei Naht aufweist.

9. Implantat gemäß einem der Ansprüche 1 bis 8, ***dadurch gekennzeichnet, dass*** das Gewirk, aus dem es besteht, aus mehreren Schichten Fäden besteht, die polymer, synthetisch oder natürlich, biokompatibel resorbierbar oder nicht sind und die miteinander verbunden sind.

## Claims

1. Implant more particularly intended for the treatment of male or female urinary incontinence, consisting of a band produced in a unitary knit having a variable width along its main dimension, and endowed - at one of its ends, at least - with a securing strap (11, 22-26) extending directly from its main structure, with the knit being procured using warp knit technology on a chain or RACHEL loom, using carbine or automatic needles, with the said needles not being supplied with one or more threads during at least one row, without interrupting the knit formation mechanism.

2. Implant in accordance with claim 1, ***characterized* in that** the mechanical properties of the band vary along the main dimension of the implant, and notably the securing straps (11, 22-26) in relation to the central area, which conventionally acts as a support zone for a subassembly.

3. Implant in accordance with claim 2, ***characterized* in that** the variable mechanical properties are the extension and the mechanical strength.

4. Implant in accordance with one of claims 1 to 3, ***characterized* in that** the lateral edges of the knit are limitatively composed of loops, to the exclusion of any free end of any of the threads of which it is composed.

5. Implant in accordance with one of claims 1 to 4, ***characterized* in that** the knit of which it is composed is procured using warp knit technology on a chain, RACHEL or hook loom, and using automatic needles.

6. Implant in accordance with claim 5, ***characterized* in that** the knit is blocked at the central support or maintaining zone (12, 22), and is extensible in the longitudinal direction and/or the crosswise direction, according to the need.

7. Implant in accordance with one of claims 1 to 6, ***characterized* in that** it incorporates at least two securing straps extending from either side of the central maintaining or support zone.

8. Implant in accordance with one of claims 1 to 8, ***characterized* in that** the knit of which it is composed is free of any stitching.

9. Implant in accordance with one of claims 1 to 8, ***characterized* in that** the knit of which it consists is composed of multiple layers of threads of synthetic polymer or absorbable or non-absorbable natural biocompatible kind, bound together.
